# EUROPÄISCHE PATENTSCHRIFT

(11) **EP 3 852 930 B1**
(45) Veröffentlichungstag und Bekanntmachung des Hinweises auf die Patenterteilung: **30.10.2024**
(21) Anmeldenummer: 19770013.1
(22) Anmeldetag: 12.09.2019
(51) Int. Cl.: B01L 9/00, C12M 1/32, G01N 1/31

(54) **ANORDNUNG ZUR DURCHFÜHRUNG VON IN-VITRO BIOKOMPATIBILITÄTSTESTS**
ASSEMBLY FOR PERFORMING IN VITRO BIOCOMPATIBILITY TESTS
SYSTÈME POUR EFFECTEUR DES ESSAIS DE BIOCOMPATIBILITÉ IN VITRO

(30) Priorität: 19.09.2018 DE 102018215956; 11.12.2018 DE 102018221415
(43) Veröffentlichungstag der Anmeldung: 28.07.2021
(73) Patentinhaber: Fraunhofer-Gesellschaft zur Förderung der angewandten Forschung e.V., 80686 München (DE)
(72) Erfinder: IßLEIB, Constantin, 01109 Dresden (DE); SPOHN, Juliane, 01109 Dresden (DE); KURZ, Susanne, 01109 Dresden (DE)
(74) Vertreter: Pfenning, Meinig & Partner mbB
(86) Internationale Anmeldenummer: PCT/EP2019/074319
(87) Internationale Veröffentlichungsnummer: WO 2020/058070

(56) Entgegenhaltungen:
- DE-A1- 10 319 712
- DE-A1- 10 319 712
- US-A1- 2004 071 605
- US-A1- 2004 071 605
- US-A1- 2005 135 974
- US-A1- 2005 135 974
- US-A1- 2007 054 273
- US-A1- 2007 054 273
- US-A1- 2008 293 157
- US-A1- 2008 293 157

## Beschreibung

Die Erfindung betrifft eine Anordnung zur Durchführung von in-vitro Biokompatibilitätstests für bevorzugt unterschiedliche mindestens einseitig planare, dichte Materialproben, die mit einer Flüssigkeit bzw. Lösung, in der biologisches Material enthalten ist, untersucht werden können. Besonders interessant sind Untersuchungen an Materialien, die in der Medizintechnik eingesetzt werden können. Dies können beispielsweise Materialien für Implantate oder beschichtete Oberflächen in der Zellkultur und für diagnostische Tests sein.

Die biologische Bewertung von festen, lösemittelundurchlässigen Testmaterialien findet grundsätzlich nach aktuellem Stand der Technik innerhalb einer Lösung statt. Dies bedeutet, dass ein Probekörper/Testmaterial in eine Mikrotiterplatte eingelegt und in eine entsprechende Lösung (z.B. Zellkulturmedium) eingebettet wird und die Lösung infolgedessen den Probekörper vollständig umgeben kann.

Betrachtet man den gesamten Probekörper kommt es zwischen verschiedenen Materialien oder auch zwischen verschiedenen Herstellern zu maßgeblichen Unterschieden in der spezifischen Oberfläche, bedingt durch die Größe und Höhe der Probekörper. Selbst innerhalb desselben Materials ist es technisch höchst anspruchsvoll zwei identische Materialproben zu erzeugen. Zusätzlich weist ein Probekörper meist nur eine "gute" Seite auf, die die Oberflächeneigenschaften des Materials repräsentiert. Die beschriebenen Unterschiede und Einflussfaktoren haben Auswirkungen auf quantifizierbare biologische Ergebnisse.

Es mangelt an einer standardisierten, wenig arbeitsaufwendigen Methodik, die ausschließlich eine definierte Oberfläche auf der guten Seite einer Probe zur biologischen Bewertung anbietet, ein für Zellkulturtests geeignetes Grundflächen-Volumenverhältnis innerhalb einer Kavität erzeugt und gleichzeitig einfach steril zu handhaben ist und gewährleistet, diese definierte Oberfläche gleichzeitig auf weitere Probekörper übertragen zu können.

Bei der biologischen Beurteilung von Materialien im Medizinproduktkontext gibt es aktuell klare Anweisungen durch die Norm DIN EN ISO 10993. Darin beschrieben ist, dass ein Probekörper/Testmaterial in eine Mikrotiterplatte eingelegt und in eine Lösung eingebettet wird und relevante Testverfahren im Gesamtvolumen der Lösung durchgeführt werden. Da eine Norm das Mindestmaß der Testung vorschreibt, war es aus Hersteller-/Prüflaborsicht bisher nicht notwendig den Testansatz zu erweitern.

Die Erfindung standardisiert den Prüfvorgang in dem für jeden Probekörper, dieselbe Oberfläche und das gleiche Volumen an Flüssigkeit einer Lösung angeboten wird und das biologische Material mit der Lösung nur Kontakt zur guten Seite des Probekörpers erhält. Biologische Ergebnisse, auch zwischen Materialien, werden somit quantifizierbar und Vergleiche werden bedeutend exakter.

In biologischen Testszenarien werden häufig Mikrotiterplatten eingesetzt. Es gibt verschiedene Formate, wie z.B. 6-Well, 12-Well, 24-Well und 96-WellPlatten. Die Wells (Kavitäten) einer Mikrotiterplatte sind alle gleich groß. Normalerweise wird die kleinstmögliche Wellgröße ausgewählt, in die die zu untersuchende Probe hineinpasst. Bei den Mikrotiterplatten ist es demzufolge nachteilig, dass unterschiedliche Proben-Geometrien oder Proben mit gleicher Geometrie, aber geringfügigen Größenunterschieden in gleichbleibenden Wellformaten geprüft werden, da es hierbei zu einer hohen, multifaktoriellen Fehlerquote kommt. Die Interaktion des biologischen Materials mit der umliegenden aus Plastik bestehenden Mikrotiterplatte wird zudem vollkommen vernachlässigt.

So sind auch Klebelösungen bekannt, bei denen Kavitäten mit einer Tiefe bis zu 2,5 mm vorhanden sind. Die Klebewirkung wird dabei beim Andrücken einer Probe freigesetzt. Sie werden nur an vergleichsweise glatten Oberflächen und häufig für zellmikroskopische Zwecke eingesetzt. Schon allein aufgrund der geringen Tiefe der Kavität ist diese Lösung für unsere Zwecke der Materialtestung nicht geeignet, da sie kein adäquates Grundfläche-Volumenverhältnis für Zellkulturansätze bietet. Ein bedeutender Nachteil könnte außerdem sein, dass das Klebemittel in das Zellkulturmedium übergeht und ggf. eine zelltoxische Wirkung hat oder mit gelöstem, biologischem Material interagiert. Weiterhin könnten Klebemittelrückstände nach der Nutzung an der Materialprobe haften bleiben, sodass die Probe im Anschluss gereinigt werden muss.

Neben Klebe- und Drucklösungen gibt es die physikalische Barrierelösung (hydrophobe Barriere), die auf der Generierung hydrophober Eigenschaften im Grenzbereich beruht. Diese hydrophoben Bereiche können einen negativen Einfluss auf das generelle Anwachsen und der Kultivierung von Zellen auf den zu prüfenden Materialoberflächen haben. Weitere Nachteile dieser Barriere-Lösungen sind eine zu geringe Höhe der Kavitäten und eine generelle Eignung nur für vergleichsweise glatte Oberflächen.

Publizierte Drucklösungen erlauben bis zu 100 µL Volumen in einer Kavität aufzunehmen. Beispielsweise drückt ein Schraubverschluss elastisches Material an einen Glasobjektträger. Gedacht sind diese Ausführungen für kleine Volumina auf Glasobjektträgern. Auf Proben, insbesondere mit unterschiedlicher Geometrie und Größe kann nicht ausreichend flexibel reagiert werden. Zudem sind mehrtägige Zellkulturversuche in diesen geringen Volumina und ohne Gewährleistung eines Gasaustausches nicht möglich.

Alle angegebenen Lösungen wurden für Glasobjektträger oder vergleichsweise glatte Oberflächen entwickelt und sind u. U. ungeeignet für andere Oberflächen insbesondere aufgrund unterschiedlicher Rauheiten. Besonders die hydrophobe Barrierelösung könnte mitunter bei rauen Oberflächen deutlich an Dichtigkeit verlieren. Außerdem sind alle aufgeführten Lösungen bei der gleichzeitigen Bearbeitung von mehreren Probekörpern sehr arbeitsintensiv. Teilweise mangelt es bei den aufgeführten Lösungen auch an der einfachen sterilen Handhabbarkeit der Probekörper mit erzeugter Kavität und eine extra "Verpackung" in Petrischalen o.ä. wird nötig.

So sind aus US 2008/0293157 A1 eine Vorrichtung und ein Verfahren zur Durchführung von Zellkulturstudien von Biomaterialien bekannt.

US 2005/0135974 A1 betrifft eine Vorrichtung zur Präparierung mehrerer Proben mit einem mehreren Arrayoberflächen.

In US 2004/0071605 A1 ist eine Analysevorrichtung für eine Vielzahl von Proben beschrieben.

In US 2007/0054273 A1 ist eine Mikroarrayvorrichtung offenbart, die beeinflussbare Reaktionsvolumina aufweist.

Es ist daher Aufgabe der Erfindung, Möglichkeiten für Untersuchungen an Proben bei der Durchführung von in-vitro Biokompatibilitätstests anzugeben, bei denen eine standardisierte Oberfläche auf Probekörpern verschiedener Rauheiten erzeugt wird, eine wenig arbeitsintensive Erzeugung dieser Kavitäten auch über mehrere Probekörper vergleichend möglich ist und eine einfache sterile Handhabbarkeit der Probekörper mit erzeugten Kavitäten gewährleistet ist.

Erfindungsgemäß wird diese Aufgabe mit einer Anordnung, die die Merkmale des Anspruchs 1 aufweist, gelöst. Vorteilhafte Ausgestaltungen und Weiterbildungen können mit in untergeordneten Ansprüchen bezeichneten Merkmalen realisiert werden.

Dabei ist auf eine Oberfläche einer Grundplatte mindestens eine Probe angeordnet. Die mindestens eine Probe kann aber auch eine Oberfläche oder einen Oberflächenbereich der Grundplatte bilden. Ein mindestens eine Durchbrechung aufweisendes Aufnahmeelement ist bevorzugt passgenau aufsetzbar.

Ein mindestens eine Durchbrechung aufweisendes Aufnahmeelement ist so auf die mindestens eine Probe aufsetzbar, dass eine in Richtung Grundplatte weisend angeordnete erste Öffnung der mindestens einen Durchbrechung, im Bereich der mindestens einen Probe angeordnet ist. Dabei bilden die mindestens eine Probe und die mindestens eine Durchbrechung den Hohlraum einer Kavität.

Auf das Aufnahmeelement ist ein Deckelelement so aufsetz- und fixierbar, dass eine Druckraft auf das Aufnahmeelement wirkt, die zu einer mindestens teilweisen Verformung des Aufnahmeelements und dem fluiddichten-Verschluss der in Richtung Grundplatte weisend angeordneten ersten Öffnung der mindestens einen Durchbrechung, führt. Während einer Untersuchung kann somit dort keine Flüssigkeit austreten.

Die Probe sollte so dimensioniert sein, dass sie mit ihrer mindestens einseitig planaren Seite die Kanten der in Richtung Grundplatte weisenden mindestens einen Durchbrechung überdeckt.

Für einen Ausgleich unterschiedlicher Probenhöhen kann man vorteilhaft platten- oder scheibenförmige Elemente mit entsprechend angepasster Dicke im Bereich einer oder mehrerer Durchbrechung(en) auf der dem Aufnahmeelement zugewandt angeordneten Oberfläche der Grundplatte zwischen Grundplatte und Materialprobe anordnen. Auf einem solchen platten- oder scheibenförmigen Element kann dann eine Probe angeordnet werden, wie dies vorab beschrieben worden ist. Damit können insbesondere Höhendifferenzen kompensiert werden. Das Einlegen von Positionierungshilfen zur einfachen Ausrichtung von Proben auf der Oberfläche der Grundplatte ist möglich.

Der Hohlraum der jeweiligen mindestens einen Durchbrechung des Aufnahmeelementes und die Probe sollten dabei mindestens eine 5 mm hohe Kavität (unverformter Zustand) bilden, wobei die innere Mantelfläche der Durchbrechung die Seitenwand und die Probe den Boden der Kavität bilden sollte.

Am Deckelelement können passende Durchbrechungen zum Aufnahmeelement vorhanden sein, über die die jeweilige Kavität mit einer Flüssigkeit, in der beispielsweise lebende Zellen enthalten sein können, befüllt werden kann. Das Deckelement kann zur einfacheren Zuordnung der Durchbrechungen mit Markierungen versehen werden.

Grundplatte und Deckelelement sind an einer Seite, bevorzugt gelenkig, miteinander verbunden und können außerdem austauschbar sein. So kann man das Deckelelement auf und zu klappen um die Anordnung zu bestücken und im geschlossenen Zustand die Untersuchungen durchführen zu können. Beim Einspannen des Aufnahmeelements zwischen Grundplatte und Deckelelement können die Druckkräfte beispielsweise mittels eines Klick-Einrast-Mechanismus als Beispiel einer formschlüssigen Verbindung ausgeübt werden. Somit kann auf adhäsive Materialien, wie z.B. Klebstoffe verzichtet werden.

An der Grundplatte und dem Deckelelement sind Verschlusselemente ausgebildet, mit denen eine formschlüssige Verbindung von Grundplatte und Deckelelement sowie eine Druckkraftausübung auf das Aufnahmeelement erreichbar sind.

Die Verschlusselemente und eine Verbindung mit der Grundplatte und Deckelelement sind miteinander verbunden, und dabei sind die entsprechenden Elemente an sich gegenüberliegend angeordneten Seiten der Anordnung angeordnet. So kann mindestens ein Gelenk, beispielsweise ein Film- oder Scharniergelenk an einer Seite und die Verschlusselemente an einer gegenüberliegenden Seite angeordnet sein.

Durch diese Art von Verbindung von Grundplatte und Deckelelement kann man auf zusätzliche Elemente, mit denen ein Verschluss einer Anordnung erreicht werden kann, verzichten, die als Einzelteile an der Anordnung angebracht werden müssen. So sind beispielsweise Schraubverbindungen kritisch wegen deren Handhabbarkeit. Außerdem erschweren zusätzliche Elemente das Sterilisieren, wenn sie nicht sogar nach jeder durchgeführten Untersuchung ausgetauscht werden müssen. Bei der erfindungsgemäßen Anordnung sind vorteilhaft keine zusätzlichen einzeln handhabbare Verbindungselemente erforderlich, mit denen die Grundplatte mit einem Deckelelement verbunden werden müssen.

Um das Verschließen und Öffnen der Anordnung zu erleichtern kann ein Griff vorhanden sein.

Das Aufnahmeelement ist aus oder mit einem elastisch verformbaren Material, das bevorzugt eine Shore Härte im Bereich 30 bis 50 aufweist, gebildet. Es kann also vollständig aus verformbarem Material, besonders bevorzugt aus einem elastisch verformbaren Material gebildet sein oder als Verbundmaterial zumindest an der Oberfläche die an der Grundplatte bzw. mindestens einer Probe anliegt mit elastisch verformbaren Material versehen sein. Dadurch kann auf zusätzliche Dichtelemente, die form- und/oder kraftschlüssig befestigt werden müssen, verzichtet werden, die ebenfalls bei einer Sterilisation nachteilig wären.

Am Deckelelement sollte mindestens eine Öffnung so angeordnet sein, dass sie eine zweite Öffnung der jeweiligen Durchbrechung des Aufnahmeelements, die in Richtung Deckelelement angeordnet ist, zumindest teilweise offen lässt.

Es kann auch ein Verschlussdeckel auf das Deckelelement aufsetzbar sein und dabei bevorzugt für eine definiert vorgebbare Positionierung und/oder Ausrichtung an einer Oberfläche von Verschlussdeckel und/oder Deckelelement mindestens ein Konstruktionselement für eine formschlüssige Ausrichtung vorhanden sein.

Ein bevorzugt zumindest teilweise optisch transparenter Verschlussdeckel kann auf die bestückte und geschlossene Anordnung aufgesetzt werden. Damit kann ein Verschluss der Deckelementöffnungen und damit die sterile Handhabbarkeit der geschlossenen Anordnung außerhalb einer Sterilwerkbank erreicht werden. Der Verschlussdeckel sollte dabei in vorgegebener Ausrichtung aufsetzbar sein und einen Gasaustausch zwischen Umgebung und Kavitäteninhalt ermöglichen.

Deckelelement und Aufnahmeelement können Durchbrechungen aufweisen, die gängigen Well-Formaten entsprechen und modular austauschbar sind. Dies trifft auch auf Deckelelement und das dazu passende Aufnahmeelement zu.

An einem Deckelelement können Elemente vorhanden sein, die einen Gasaustausch mit der Umgebungsatmosphäre ermöglichen. Dies kann mindestens eine Öffnung sein. Eine Öffnung kann auch mit einer Membran versehen sein, die gasdurchlässig sein sollte. Eine Membran kann dabei vorteilhaft auch für mindestens ein ausgewähltes Gas oder Gasgemisch selektiv durchlässig sein.

Das Aufnahmeelement sollte in der Grundplatte und/oder dem Deckelelement formschlüssig gehalten sein. Dadurch kann eine definiert positionierte Fixierung von Durchbrechungen in Bezug zur Anordnung jeweiliger Proben erreicht werden.

Mehrere Durchbrechungen können in zugeordneten Gruppen ausgebildet bzw. angeordnet sein. So kann je nach Größe der Probe eine Gruppe von Durchbrechungen in einem Aufnahmeelement für jeweils eine Probe verwendet werden. Es können auf mehreren Proben gleiche Gruppen von Kavitäten erzeugt werden. Dies ermöglicht es Mehrfachbestimmungen auf einer Probe und Mehrfachbestimmungen über mehrere Proben durchzuführen. Ebenfalls können verschiedene Untersuchungen auf einer Probe parallel durchgeführt werden.

Es können auch mehrere unterschiedliche Proben auf einer Oberfläche einer Grundplatte angeordnet sein oder die unterschiedlichen Proben können jeweils eine Oberfläche oder einen Oberflächenbereich der Grundplatte bilden. An der Grundplatte kann mindestens eine Öffnung, für ein Ein- und Ausführen von Objekten, insbesondere Objektträgern, vorhanden sein. Durch eine solche Öffnung können Objekte von außen in die Anordnung spezifisch eingeschoben, darin bewegt und wieder entfernt werden. Mindestens eine an der Öffnung vorhandene Kante kann das Ein- und Ausführen eines Objektes in bzw. aus der Anordnung erleichtern.

Als standardisierte Oberflächen wurden bisher die in der Molekular-/Zellbiologie üblichen Mikrotiterplatten-Formate gewählt. Mikrotiterplatten nutzen kleine, definierte, runde Kavitäten um molekularbiologische Tests standardisiert durchzuführen. Somit besteht ein sehr guter Bezugspunkt bzw. Vergleich zu den bereits etablierten biologischen Bewertungsmethoden im Labor. Vorteilhaft können Aufnahmeelemente bei der Erfindung eingesetzt werden, deren Format herkömmlichen Mikrotiterplatten entspricht bzw. ihnen nahe kommt.

Mit der Erfindung kann ein abgegrenzter, wasserdichter Raum mit standardisiertem Oberflächenareal mit mindestens einer Durchbrechung für mindestens eine Probe bzw. Probekörper zur Verfügung gestellt werden. Die Höhe der dadurch entstehenden Kavitäten beträgt ≥ 5 mm. Durch die Erzeugung standardisierter bzw. gleicher Oberflächen auf den Proben und dem angepassten Flüssigkeitsvolumen in diesen Kavitäten kann eine biologische Bewertung von Proben weitaus genauere Aussagen treffen als der Stand der Technik es bisher zulässt. Kanten, Wände sowie Rückseiten von Durchbrechungen mit den entsprechenden Oberflächen von Grundplatte und Deckelelement und damit zusammenhängende Fehlerquellen, in Hinsicht auf quantifizierbare biologische Ergebnisse können durch die Erfindung systematisch vermieden werden.

Durch eine Anpressdruck-Lösung bedarf es keiner Klebstoffe oder ähnlichen Substanzen, die im Kontakt mit Lösungen in die Flüssigkeiten diffundieren. Außerdem bleibt somit das Aufnahmeelement vollständig unbehandelt, da das elastische Material keine Rückstände auf einer Probe bzw. einem Probekörper zurücklässt. Das Aufnahmeelement besteht bevorzugt aus einem biokompatiblen und ggf. auch einem chemisch inerten Material, sodass Interaktionen mit Lösungsmitteln oder biologischem Material während des Versuchszeitraums minimiert werden können. Weiterhin kann die Erfindung verlässlich lösemitteldicht, auch bei rauen Oberflächen ausgebildet sein, da sich das elastische Material leicht an die Oberflächen anpresst.

Die Erfindung kann eingesetzt werden, um Proben/Probekörper unterschiedlichster Materialkompositionen und Dimensionen standardisiert in biologischem Kontext zu vergleichen. Besonders die Materialtestung im Bereich der Medizintechnik (u.a. auch Implantate) bietet ein umfangreiches Spektrum an Materialkompositionen und Materialeigenschaften, für die eine derartige Vergleichsmethode, die repräsentative und reproduzierbare Ergebnisse liefert, dringend erforderlich ist.

Mit der Erfindung können auch biologische Untersuchungen mit Proben, die mit einer Beschichtung aus einem Material, degradierbaren Materialien und Materialbeschichtungen, bei denen die beschriebene Anordnung adaptiert werden kann, um die relative Fluiddichte während des Versuchs zu gewährleisten zu können, durchgeführt werden.

Die biologische Beurteilung von Materialien kann durch die Erfindung in Routine-Laboren genutzt/angeboten werden. Es können zukünftig auch patienten-spezifische Reaktionen auf verschiedene Materialien getestet werden, wodurch Materialzusammensetzungen kundenspezifisch angeboten werden können.

In der biologischen sowie materialwissenschaftlichen Forschung bietet die Erfindung einen immensen Anwendungsbereich, da grundsätzlich alle für den Zeitraum der Untersuchung dichten Materialien standardisiert biologisch untersucht werden können. Unter Zuhilfenahme der erfindungsgemäßen Anordnung können die für die Mikrotiterplatten etablierten molekularbiologischen Assays direkt auf die Materialtestung übertragen werden und anhand der resultierenden quantitativen Messdaten kann ein direkter Vergleich zwischen Material und Mikrotiter-Plastik als Referenz durchgeführt werden.

Im Bereich der biologischen Testung von Medizinprodukten (ISO 10993) könnten durch Einsatz der Erfindung neue quantitative Methoden eingesetzt werden, die ohne Erzeugung einer standardisierten Oberfläche auf der guten Seite eines Probekörpers nicht möglich wären.

Nachfolgend soll die Erfindung beispielhaft näher erläutert werden.

Dabei zeigen:
Figur 1 a-c ein Beispiel einer Grundplatte mit einem passenden Aufnahmeelement sowie eine Probe in perspektivischer Darstellung;
Figur 2 eine perspektivische Darstellung einer Grundplatte mit eingesetzter Probe;
Figur 3 eine perspektivische Darstellung einer Grundplatte mit eingesetzter Probe und aufgesetztem Aufnahmeelement;
Figur 4 ein Beispiel eines Deckelelements, das bei der erfindungsgemäßen Anordnung eingesetzt werden kann;
Figur 5 eine Zusammenbauzeichnung eines Beispiels einer erfindungsgemäßen Anordnung;
Figur 6 ein Beispiel für einen Verschlussdeckel und
Figur 7 einen Verschlussdeckel, der auf einer Anordnung bestehend aus Grundplatte, Probe mit Aufnahmeelement und Deckelement aufgesetzt ist.

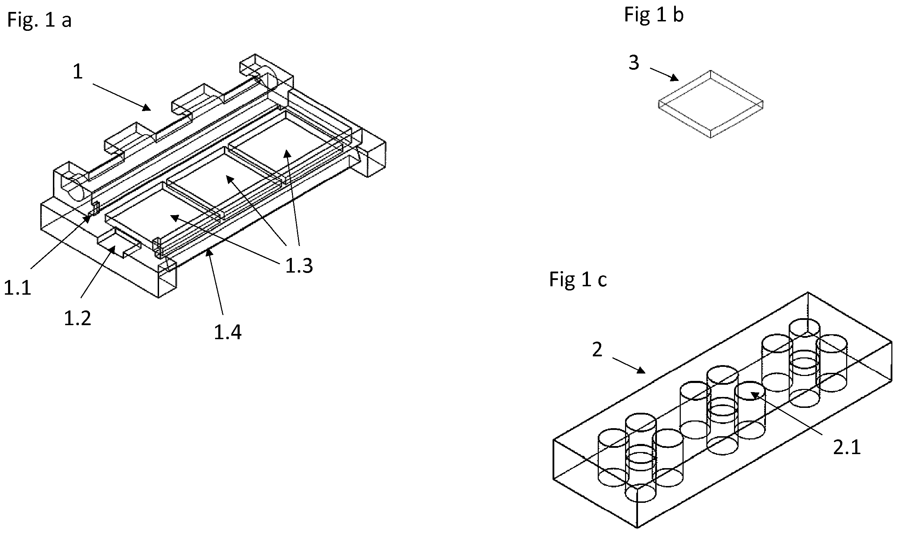

In Figur 1a ist ein Beispiel einer Grundplatte 1 gezeigt. Dabei sind in der Grundplatte 1 Aufnahmen 1.3 für einen formschlüssigen Halt von Proben 3 vorhanden.

Außerdem sind eine spaltförmige Öffnung 1.1 für ein Ein- und Ausführen von Objekten, insbesondere Objektträgern, und eine Kante 1.2, die diesen Vorgang vereinfachen soll, vorhanden

Figur 1b zeigt ein Beispiel einer Probe 3, die auf eine Oberfläche der Grundplatte 1 aufgelegt werden kann. Dies erfolgt bevorzugt im Bereich einer Aufnahme 1.3.

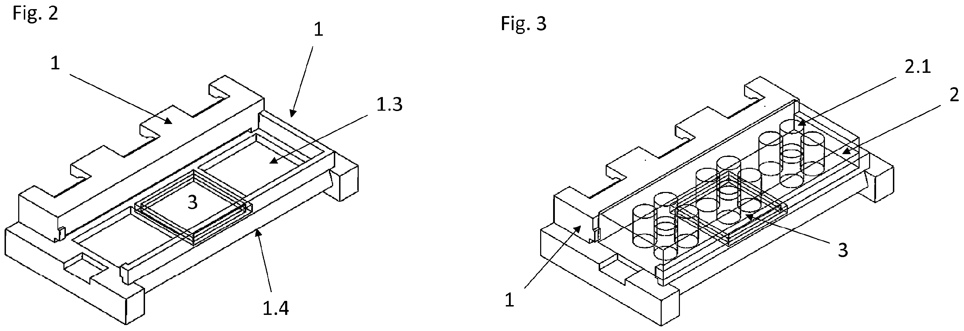

Nach dem Einsetzen mindestens einer Probe 3 kann ein in Figur 1c gezeigtes Aufnahmeelement 2 auf die Grundplatte 1 aufgesetzt werden. Am Aufnahmeelement 2 sind Durchbrechungen 2.1 vorhanden und so angeordnet, dass sie mit einem Oberflächenbereich einer Probe 3 kommunizieren und damit eine Kavität bilden können, die mit einer Flüssigkeit, in der insbesondere biologisches Material enthalten ist, befüllt werden kann, um die jeweilige Probe 3 in biologischen oder biochemischen Szenarios untersuchen zu können. Dieser Aufbau ist in Figur 3 gezeigt.

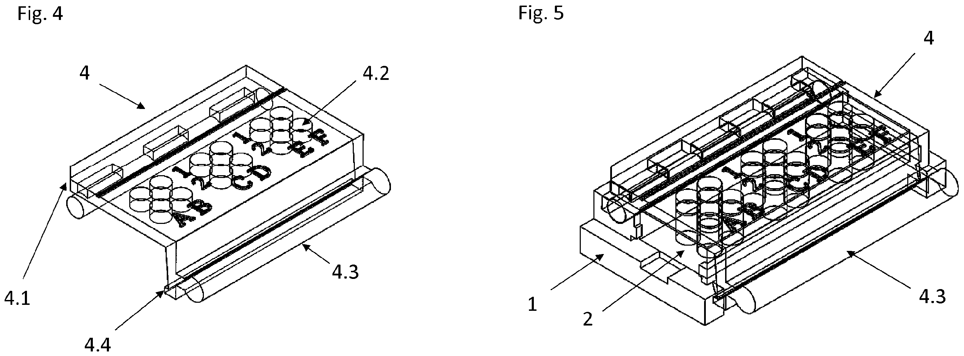

Das in Figur 4 gezeigte Deckelelement 4 kann mit der Grundplatte 1 an einer Stirnseite gelenkig verbunden sein. In dem Deckelelement 4 sind bei diesem Beispiel ebenfalls Durchbrechungen 4.2 vorhanden und so angeordnet, dass sie mit den Proben 3 und den Durchbrechungen 2.1 in dem Aufnahmeelement 2 kommunizieren, so dass Befüllen und Entnahme von Flüssigkeiten in die Durchbrechungen 2.1 und ein Gasaustausch möglich ist. Zusätzlich kann man den Inhalt der Kavitäten visuell überwachen. An der Stirnseite des Deckelelements 4, die der gelenkigen Verbindung gegenüberliegend angeordnet ist, ist eine Kante 4.4 ausgebildet, die im zusammengesetzten Zustand eine Unterkante 1.4 der Grundplatte 1 für eine formschlüssige Verbindung hintergreifen kann. Dadurch können Druckkräfte auf das elastisch verformbare Aufnahmeelement 2 ausgeübt werden, die ein Zusammenpressen und einen fluiddichten Verschluss der Kavitäten bewirken. Das Aufnahmeelement 2 wird dabei elastisch verformt. An dieser Stirnkante kann zur Erleichterung ein Griffelement 4.3 vorhanden sein, mit dem ein Öffnen und Verschließen der Anordnung erleichtert werden kann.

Figur 5 zeigt einen zusammengebauten geschlossenen Zustand.

Am Deckelelement 4 können Markierungen oder Kennzeichnungselemente, beispielsweise in Form von Buchstaben und/oder Zahlen vorhanden sein, mit denen die Zuordnung und Erkennung bestimmter Proben 3 erleichtert werden kann.

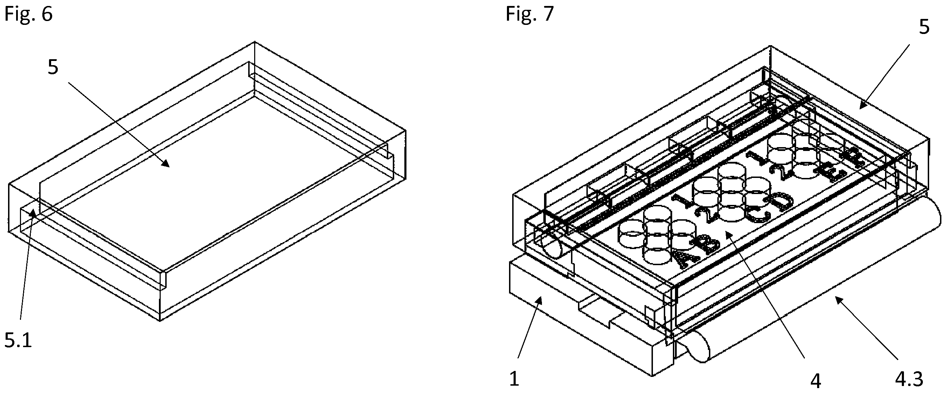

Der in Figur 6 gezeigte Verschlussdeckel 5 kann auf das Deckelelement 4 aufgesetzt und an dem Deckelelement 4 und/oder der Grundplatte 1 formschlüssig befestigt werden, wie dies in Figur 7 gezeigt ist. Der Verschlussdeckel 5 weist dazu eine umlaufende Kante 5.1 auf, die im Zusammenspiel mit der Kante 4.1 des Deckelelements 4 das Aufsetzen auf das Deckelelement 4 in einer vorgegebenen Ausrichtung ermöglicht.

Der Verschlussdeckel 5 sollte zumindest in dem Bereich, der Durchbrechungen 2.1 sowie 4.2 überdeckt, optisch transparent sein.

Das Aufnahmeelement 2 kann beispielsweise aus einem Silikon bestehen und eine Dimensionierung aufweisen, wie sie im allgemeinen Teil der Beschreibung genannt worden ist.

Die Oberfläche der Grundplatte 1 auf der das Aufnahmeelement 2 aufgesetzt ist, weist stegförmige Erhebungen auf, an denen sich äußere Kanten des Aufnahmeelements 2 anlegen können und so ein formschlüssiger Halt des Aufnahmeelements 2 auf der Grundplatte 1 erreicht werden kann.

Auch am Deckelelement 4 können stegförmige Erhebungen in Richtung Aufnahmeelement 2 ausgebildet sein, mit denen ein formschlüssiger Halt des Aufnahmeelements 2 erreicht werden kann.

An einer Stirnseite können Grundplatte 1 und Deckelelement 4 gelenkig miteinander verbunden sein, so dass man das Deckelelement 4 auf- und zu klappen kann. An der gegenüberliegenden Stirnseite können an Grundplatte 1 und Deckelelement 4 Verschlusselemente ausgebildet sein. Sie können eine Art Klipp- bzw. Schnappverschluss bilden, wenn das Deckelelement 4 die Anordnung verschließt.

Die Verschlusselemente können so ausgebildet, dimensioniert und angeordnet sein, dass beim Zuklappen des Deckelelements 4 und dem formschlüssigen Eingriff der Verschlusselemente zwischen Grundplatte 1 und Deckelelement 4 die Druckkräfte auf das Aufnahmeelement 2 wirken, mit denen die erforderliche Verformung, die zum Verschließen der Öffnungen von Durchbrechungen 2.1, die an der Oberfläche der jeweiligen Probe 3 angeordnet sind, führen.

Mit den zwischen Grundplatte 1 und Deckelelement 4 auf das Aufnahmeelement 2 und die dem Aufnahmeelement 2 zugewandte Oberfläche der Probe(n) 3 wirkenden Druckkräfte kann bei entsprechend verformtem Aufnahmeelement 2 eine fluiddichte Abdichtung zwischen der Oberfläche des Aufnahmeelements 2 und der diesem zugewandten Oberfläche der Probe(n) 3 im Bereich der Kavitäten erreicht werden. Dazu sollten die jeweilige Flächengrö-ßen der Probe(n) 3 im Bereich der ersten Öffnungen und die Gestaltung in diesem Bereich so dimensioniert und gewählt sein, dass keine Flüssigkeit austreten kann.

Am Deckelelement 4 sind Öffnungen 4.2 vorhanden, die so dimensioniert und angeordnet sind, dass zweite Öffnungen von Durchbrechungen 2.1, die am Deckelelement 4 angeordnet sind frei gehalten sind, so dass ein Gasaustausch mit der Umgebung erfolgen kann. Die mit den Durchbrechungen 2.1 und der/den Probe(n) 3 gebildeten Kavitäten können über die zweiten Öffnungen, die an der den ersten Öffnungen gegenüberliegenden Oberfläche des Aufnahmeelements 2, also an der dem Deckelelement 4 zugewandten Seite angeordnet sind, mit biologischem Material und Lösungsmittel befüllt werden, bevor der jeweilige Test durchgeführt wird. Während oder nach einem Test kann Flüssigkeit durch die Durchbrechungen 2.1 und 4.2 ebenfalls entnommen werden, sodass auch Waschschritte der Kavitäten möglich sind.

Grundplatte 1, Deckelelement 4 und Verschlussdeckel 5 sind bei diesem Beispiel aus autoklavierbarer Plastik (Polycarbonat) gebildet.

## Patentansprüche

1. Anordnung zur Durchführung von in-vitro Biokompatibilitätstests mit einem Aufnahmeelement (2) und einem Deckelelement (4), bei der mindestens eine Probe (3) auf einer Oberfläche einer Grundplatte (1) angeordnet ist oder bei der die mindestens eine Probe (3) eine Oberfläche oder einen Oberflächenbereich der Grundplatte (1) bildet und das mindestens eine Durchbrechung (2.1) aufweisende Aufnahmeelement (2) so auf die mindestens eine Probe (3) aufsetzbar ist, dass eine in Richtung Grundplatte (1) weisend angeordnete erste Öffnung der mindestens einen Durchbrechung (2.1), im Bereich der mindestens einen Probe (3) angeordnet ist, wobei
die mindestens eine Durchbrechung (2.1) mit ihrem Hohlraum und die Probe (3) eine Kavität bilden, und
das Aufnahmeelement (2) aus oder mit einem elastisch verformbaren Material gebildet ist; und
auf das Aufnahmeelement (2) das Deckelelement (4) so aufsetz- und fixierbar ist, dass eine Druckkraft auf das Aufnahmeelement (2) wirkt, die zu einer mindestens teilweisen Verformung des Aufnahmeelements (2) und dem fluiddichten-Verschluss der in Richtung Grundplatte (1) weisend angeordneten ersten Öffnung der mindestens einen Durchbrechung (2.1), führt; und
an der Grundplatte (1) und an dem Deckelelement (4) Verschlusselemente (1.4, 4.4) ausgebildet sind, um eine formschlüssige Verbindung von Grundplatte (1) und Deckelelement (4) sowie eine Druckkraftausübung auf das Aufnahmeelement (2) zu erreichen, wobei
die Verschlusselemente (1.4, 4.4) und eine Verbindung zwischen der Grundplatte (1) und dem Deckelelement (4) miteinander verbunden und an sich gegenüberliegend angeordneten Seiten der Anordnung angeordnet sind.

2. Anordnung nach Anspruch 1, **dadurch gekennzeichnet, dass** das Aufnahmeelement (2) aus einem biokompatiblen und/oder sterilisierbaren Material gebildet ist.

3. Anordnung nach einem der vorhergehenden Ansprüche, **dadurch gekennzeichnet, dass** das Aufnahmeelement (2) aus oder mit einem elastisch verformbaren Material, das eine Shore Härte im Bereich 30 bis 50 aufweist, gebildet ist.

4. Anordnung nach einem der vorhergehenden Ansprüche, **dadurch gekennzeichnet, dass** die mindestens eine Probe (3) auf der Oberfläche der Grundplatte (1) formschlüssig fixierbar ist.

5. Anordnung nach einem der vorhergehenden Ansprüche, **dadurch gekennzeichnet, dass** am Deckelelement (4) mindestens eine Öffnung (4.2) so angeordnet ist, dass sie eine zweite Öffnung der jeweiligen Durchbrechung (2.1) des Aufnahmeelements (2), die in Richtung Deckelelement (4) angeordnet ist, zumindest teilweise offen lässt.

6. Anordnung nach einem der vorhergehenden Ansprüche, **dadurch gekennzeichnet, dass** die Grundplatte (1) und das Deckelement (4) an einer Seite mit mindestens einem Gelenk miteinander verbunden sind.

7. Anordnung nach einem der vorhergehenden Ansprüche, **dadurch gekennzeichnet, dass** entsprechend der in der Zellkulturforschung gängigen Well-Formate, das Deckelelement (4) und das dazu passende Aufnahmeelement (2) modular austauschbar sind.

8. Anordnung nach einem der vorhergehenden Ansprüche, **dadurch gekennzeichnet, dass** die mindestens eine Probe (3) in einer Kavität (1.3), die in der Grundplatte (1) ausgebildet ist, einsetzbar ist und nach Auflegen des Aufnahmeelements (2) eine oder mehrere Durchbrechungen (2.1) im Bereich einer Probe (3) angeordnet sind und die jeweilige Probe (3) dabei nicht die Innenwand der anliegenden Durchbrechungen (2.1) des Aufnahmeelements (2) berührt.

9. Anordnung nach einem der vorhergehenden Ansprüche, **dadurch gekennzeichnet, dass** mehrere Durchbrechungen (2.1) in zugeordneten Gruppen ausgebildet oder angeordnet sind.

10. Anordnung nach einem der vorhergehenden Ansprüche, **dadurch gekennzeichnet, dass** das Aufnahmeelement (2) in der Grundplatte (1) und/oder dem Deckelelement (4) formschlüssig gehalten ist.

11. Anordnung nach einem der vorhergehenden Ansprüche, **dadurch gekennzeichnet, dass** ein Verschlussdeckel (5) auf das Deckelelement (4) aufsetzbar ist, einen Gasaustausch mit der Umgebung erlaubt und bevorzugt für eine definiert vorgebbare Positionierung und/oder Ausrichtung an einer Oberfläche von Verschlussdeckel (5) und/oder Deckelelement (4) mindestens ein Konstruktionselement (4.1, 5.1) für eine formschlüssige Ausrichtung vorhanden ist.

12. Anordnung nach einem der vorhergehenden Ansprüche, **dadurch gekennzeichnet, dass** an der Grundplatte (1) mindestens eine Öffnung (1.1), für ein Ein- und Ausführen von Objekten, insbesondere Objektträgern, vorhanden ist.

13. Anordnung nach einem der vorhergehenden Ansprüche, **dadurch gekennzeichnet, dass** am Deckelelement (4) und/oder dem Verschlussdeckel (5) Elemente vorhanden sind, die einen Gasaustausch mit der Umgebungsatmosphäre ermöglichen.

14. Anordnung nach einem der vorhergehenden Ansprüche, **dadurch gekennzeichnet, dass** mehrere unterschiedliche Proben (3) auf einer Oberfläche einer Grundplatte (1) angeordnet sind oder die unterschiedlichen Proben (3) jeweils eine Oberfläche oder einen Oberflächenbereich der Grundplatte (1) bilden.

## Claims

1. An assembly for performing in-vitro biocompatibility tests with a receiving element (2) and a cover element (4), in which at least one sample (3) is arranged on a surface of a base plate (1) or in which the at least one sample (3) forms a surface or a surface area of the base plate (1) and the receiving element (2) having at least one through-hole (2.1) can be placed on the at least one sample (3) such that a first opening, which is disposed pointing in the direction of the base plate (1), of the at least one through-hole (2.1) is arranged in the region of the at least one sample (3), wherein
the at least one through-hole (2.1) with its hollow area and the sample (3) form a cavity, and
the receiving element (2) is made from or with an elastically deformable material; and
the cover element (4) can be placed and fixed onto the receiving element (2) such that a pressure force acts on the receiving element (2), which leads to an at least partial deformation of the receiving element (2) and the fluid-tight closure of the first opening, which is disposed pointing in the direction of the base plate (1), of the at least one through-hole (2.1); and
closure elements (1.4, 4.4) are formed on the base plate (1) and on the cover element (4) in order to achieve a positive connection between the base plate (1) and the cover element (4) and to exert a compressive force on the receiving element (2), wherein the closure elements (1.4, 4.4) and a connection between the base plate (1) and the cover element (4) are connected to one another and are arranged on opposite sides of the arrangement.

2. The assembly according to claim 1, **characterized in that** the receiving element (2) is made from a biocompatible and/or sterilizable material.

3. The assembly according to any one of the preceding claims, **characterized in that** the receiving element (2) is made from or with an elastically deformable material which has a Shore hardness in the range of 30 to 50.

4. The assembly according to any one of the preceding claims, **characterized in that** the at least one sample (3) can be fixed positively on the surface of the base plate (1).

5. The assembly according to any one of the preceding claims, **characterized in that** at least one opening (4.2) is arranged on the cover element (4) such that it leaves at least partially open a second opening of the respective through-hole (2.1) of the receiving element (2) which is arranged in the direction of the cover element (4).

6. The assembly according to any one of the preceding claims, **characterized in that** the base plate (1) and the cover element (4) are connected to one another on one side by at least one joint.

7. The assembly according to any one of the preceding claims, **characterized in that** according to the current well formats in cell culture research, the cover element (4) and the receiving element (2) adapted thereto are exchangeable in a modular manner.

8. The assembly according to any one of the preceding claims, **characterized in that** the at least one sample (3) can be inserted in a cavity (1.3) which is formed in the base plate (1) and, after placing the receiving element (2) one or more through-holes (2.1) are provided in the region of a sample (3) and the respective sample (3) does not thereby contact the inner wall of the adjacent through-holes (2.1) of the receiving element (2).

9. The assembly according to any one of the preceding claims, **characterized in that** a plurality of through-holes (2.1) are formed or arranged in associated groups.

10. The assembly according to any one of the preceding claims, **characterized in that** the receiving element (2) is retained positively in the base plate (1) and/or the cover element (4).

11. The assembly according to any one of the preceding claims, **characterized in that** a closure cover (5) can be placed onto the cover element (4), allows gas exchange with the environment and, preferably for a defined predeterminable positioning and/or orientation on a surface of the closure cover (5) and/or cover element (4), at least one construction element (4.1, 5.1) is provided for a positive orientation.

12. The assembly according to any one of the preceding claims, **characterized in that** on the base plate (1), at least one opening (1.1) is provided for guiding objects in and out, in particular object carriers.

13. The assembly according to any one of the preceding claims, **characterized in that** elements which allow gas exchange with the ambient atmosphere are provided on the cover element (4) and/or on the closing cover (5).

14. The assembly according to any one of the preceding claims, **characterized in that** a plurality of different samples (3) are arranged on a surface of a base plate (1) or the various samples (3) respectively form a surface or a surface area of the base plate (1).

## Revendications

1. Système permettant de mettre en oeuvre des essais de biocompatibilité in vitro, comprenant un élément de réception (2) et un élément formant couvercle (4), dans lequel au moins un échantillon (3) est placé sur une surface d'une plaque de base (1) ou dans lequel le au moins un échantillon (3) forme une surface ou une région de surface de la plaque de base (1) et l'élément de réception (2), qui présente au moins une perforation (2.1), peut être placé sur le au moins un échantillon (3) de sorte qu'une première ouverture, orientée dans la direction de la plaque de base (1), de la au moins une perforation (2.1) est agencée dans la région du au moins un échantillon (3), dans lequel
la au moins une perforation (2.1) et son espace vide forment avec l'échantillon (3) une cavité, et
l'élément de réception (2) est formé à partir d'un matériau élastiquement déformable ou comprend celui-ci ; et
l'élément formant couvercle (4) peut être placé ou immobilisé sur l'élément de réception (2) de sorte qu'une force de compression agit sur l'élément de réception (2) et conduit à une déformation au moins partielle de l'élément de réception (2) et à la fermeture étanche aux fluides de la première ouverture, orientée en direction de la plaque de base (1), de la au moins une perforation (2.1) ; et
des éléments de fermeture (1.4, 4.4) sont formés au niveau de la plaque de base (1) et de l'élément formant couvercle (4) afin d'obtenir une liaison par complémentarité de forme entre la plaque de base (1) et l'élément formant couvercle (4) ainsi que l'exercice d'une force de compression sur l'élément de réception (2), dans lequel les éléments de fermeture (1.4, 4.4) et une liaison entre la plaque de base (1) et l'élément formant couvercle (4) sont reliés l'un à l'autre et sont agencés sur des côtés opposés du système.

2. Système selon la revendication 1, **caractérisé en ce que** l'élément de réception (2) est formé à partir d'un matériau biocompatible et/ou stérilisable.

3. Système selon l'une quelconque des revendications précédentes, **caractérisé en ce que** l'élément de réception (2) est formé à partir d'un matériau élastiquement déformable présentant une dureté Shore située dans la plage comprise entre 30 et 50 ou comprend celui-ci.

4. Système selon l'une quelconque des revendications précédentes, **caractérisé en ce que** le au moins un échantillon (3) peut être immobilisé par complémentarité de forme sur la surface de la plaque de base (1).

5. Système selon l'une quelconque des revendications précédentes, **caractérisé en ce qu'**au moins une ouverture (4.2) est agencée au niveau de l'élément formant couvercle (4) de manière à laisser au moins partiellement ouverte une seconde ouverture de la perforation (2.1) respective, agencée en direction de l'élément formant couvercle (4), de l'élément de réception (2).

6. Système selon l'une quelconque des revendications précédentes, **caractérisé en ce que** la plaque de base (1) et l'élément formant couvercle (4) sont reliés l'un à l'autre sur un côté par au moins une articulation.

7. Système selon l'une quelconque des revendications précédentes, **caractérisé en ce que** l'élément formant couvercle (4) et l'élément de réception (2) lui correspondant peuvent être remplacés de manière modulaire en se conformant aux formats de puits usuels dans la recherche en culture cellulaire.

8. Système selon l'une quelconque des revendications précédentes, **caractérisé en ce que** le au moins un échantillon (3) peut être inséré dans une cavité (1.3) formée dans la plaque de base (1) et une ou plusieurs perforation(s) (2.1) est/sont agencée(s) dans la région d'un échantil-Ion (3) après la mise en place de l'élément de réception (2), et l'échantillon (3) respectif n'est pas en contact avec la paroi intérieure des perforations (2.1) adjacentes de l'élément de réception (2).

9. Système selon l'une quelconque des revendications précédentes, **caractérisé en ce que** plusieurs perforations (2.1) sont formées ou agencées dans des groupes dédiés.

10. Système selon l'une quelconque des revendications précédentes, **caractérisé en ce que** l'élément de réception (2) est retenu par complémentarité de forme dans la plaque de base (1) et/ou dans l'élément formant couvercle (4).

11. Système selon l'une quelconque des revendications précédentes, **caractérisé en ce qu'**un couvercle de fermeture (5) peut être placé sur l'élément formant couvercle (4) et permet un échange gazeux avec l'environnement, et au moins un élément de construction (4.1, 5.1) destiné à un alignement avec complémentarité de forme est de manière préférée présent en vue d'un positionnement et/ou d'un alignement pouvant être prédéfini du couvercle de fermeture (5) et/ou de l'élément formant couvercle (4) au niveau d'une surface.

12. Système selon l'une quelconque des revendications précédentes, **caractérisé en ce qu'**au moins une ouverture (1.1) destinée à l'introduction et à la mise en oeuvre d'objets, en particulier de porte-objets, est présente sur la plaque de base (1).

13. Système selon l'une quelconque des revendications précédentes, **caractérisé en ce que** des éléments permettant un échange de gaz avec l'atmosphère ambiante sont présents sur l'élément formant couvercle (4) et/ou sur le couvercle de fermeture (5).

14. Système selon l'une quelconque des revendications précédentes, **caractérisé en ce que** plusieurs échantillons (3) différents sont agencés sur une surface d'une plaque de base (1) ou bien les échantillons (3) différents forment respectivement une surface ou une région de surface de la plaque de base (1).
